# EUROPEAN PATENT APPLICATION

(11) **EP 4 574 129 A1**
(43) Date of publication of application: **25.06.2025**
(21) Application number: 24213853.5
(22) Date of filing: 19.11.2024
(51) Int. Cl.: A61K 8/04, A61K 8/25, A61K 8/26, A61K 8/39, A61Q 1/02

(54) **COSMETIC COMPOSITION COMPRISING NATURAL MATERIAL**

(30) Priority: 23.11.2023 KR 20230164911
(71) Applicant: Cosmax, Inc., Hwaseong-si, Gyeonggi-do 18622 (KR)
(72) Inventor: YOO, Eun Kyung, 13486 Gyeonggi-do (KR); AHN, Youjin, 13486 Gyeonggi-do (KR); LEE, Wha Young, 13486 Gyeonggi-do (KR); PARK, Myeong Sam, 04987 Seoul (KR)
(74) Representative: Mammel und Maser Patentanwälte PartG mbB

(57) **Abstract**

Disclosed herein is a cosmetic composition comprising a polyglyceryl-based emulsifier, an oil-phase thickener, a water-phase thickener, and a pigment. The cosmetic composition according to the present disclosure includes natural materials and provides excellent sensation of use and color durability without issues such as stiffness, greasiness, or darkening. Additionally, the cosmetic composition offers high pigment dispersibility and emulsion stability.

## Description

### CROSS-REFERENCE TO RELATED APPLICATION(S)

This application is based on and claims priority under 35 U.S.C. 119 to Korean Patent Application No. 10-2023-0164911, filed on November 23, 2023, in the Korean Intellectual Property Office, the disclosure of which is herein incorporated by reference in its entirety.

### BACKGROUND OF THE INVENTION

### 1. Field of the invention

The present disclosure relates to a cosmetic composition and, more specifically, to a foundation cosmetic composition comprising natural material.

### 2. Description of the Prior Art

Conventional oil-in-water (O/W) foundation cosmetic compositions have used PEG-based or silicone-based emulsifiers to achieve excellent emulsion stability and superior makeup effects. In addition to emulsifiers, synthetic materials represented by PEG-based and silicone-based substances have been used for thickeners, oils, and pigments. These synthetic emulsifiers, thickeners, oils, and pigments create a stable emulsion formulation and play a significant role in the feel and color durability of the foundation.

However, with the increasing environmental awareness, there has been a rapid rise in demand for natural cosmetics that do not use synthetic materials. Consequently, there is active development of cosmetic compositions that limit the use of synthetic materials and include natural materials. Nonetheless, excluding PEG-based and silicone-based materials, which are typical synthetic substances, poses many challenges in achieving the stability, sensation of use, and color representation of the foundation.

### [Related Art Document]

### [Patent Literature]

(Patent Literature 1) KR 10-2018-0082641 A

### SUMMARY OF THE INVENTION

To solve the aforementioned problems encountered in the related art, the present disclosure aims to provide a foundation cosmetic composition including natural materials.

An aspect of the present disclosure provides a cosmetic composition including a polyglyceryl-based emulsifier, an oil-phase thickener, a water-phase thickener, and a pigment.

The polyglyceryl-based emulsifier may include one or more selected from the group consisting of polyglyceryl pentaisostearate, polyglyceryl polyricinoleate, polyglyceryl-5 laurate, polyglyceryl-2 caprylate, and polyglyceryl-6 pentaoleate. Additionally, the polyglyceryl-based emulsifier may be contained in an amount of 0.1 to 20% by weight, based on the total weight of the composition.

The oil-phase thickener may include one or more selected from the group consisting of hectorite and bentonite. Additionally, the oil-phase thickener may be contained in an amount of 0.1 to 5% by weight, based on the total weight of the composition.

The water-phase thickener may include one or more selected from the group consisting of hectorite, bentonite, xanthan gum, magnesium silicate, magnesium aluminum silicate, lithium magnesium silicate, and magnesium sodium silicate. Additionally, the water-phase thickener may be included in an amount of 0.1 to 5% by weight, based on the total weight of the composition.

The pigment may include one or more selected from the group consisting of titanium dioxide, zinc oxide, yellow iron oxide, red iron oxide, and black iron oxide. Additionally, the pigment may be contained in an amount of 0.1 to 50% by weight, based on the total weight of the composition. The pigment may also be coated with one or more selected from the group consisting of lauroyl lysine, lysine, sodium lauroyl glutamate, glutamate, and disodium stearoyl glutamate.

The cosmetic composition may have a viscosity of 3,000 to 30,000 cps. Additionally, the cosmetic composition may have a hardness of 10 to 30 dyne/cm².

According to one embodiment of the present disclosure, a stable cosmetic composition without using silicone-based or PEG-based emulsifiers can be provided.

Furthermore, according to one embodiment of the present disclosure, a foundation cosmetic composition with excellent feel and color durability can be provided.

### DETAILED DESCRIPTION OF THE EXEMPLARY EMBODIMENTS

Hereinafter, the present disclosure will be described in more detail with reference to examples. However, these examples are provided to illustrate the present disclosure and should not be construed as limiting the scope of the present disclosure.

According to an aspect thereof, the present disclosure provides a cosmetic composition comprising a polyglyceryl-based emulsifier, an oil-phase thickener, a water-phase thickener, and a pigment.

The cosmetic composition of the present disclosure may have a viscosity of 3,000 to 30,000 cps. In the present disclosure, the viscosity is measured using a Brookfield viscometer under conditions of 64 spindle, 12 rpm, 1 minute, and 25°C. Given a viscosity of less than 3,000 cps, the composition exhibits low viscosity stability. A viscosity exceeding 30,000 cps makes it difficult to dispense the composition from the container.

The cosmetic composition of the present disclosure may have a hardness of 10 to 30 dyne/cm². In the present disclosure, the hardness is measured using a CR-3000EX-L hardness tester (SUN SCIENTIFIC, Japan) under conditions of 9 spindle and 2 cm/min speed. If the hardness is less than 10 dyne/cm², the composition exhibits low hardness stability. If the hardness exceeds 30 dyne/cm², the composition becomes poor in spreadability.

Containing both oil-phase and water-phase thickeners, the cosmetic composition provided in the present disclosure retains excellent stability within the above viscosity range.

The cosmetic composition may further comprise oil, purified water, and other additives.

The cosmetic composition of the present disclosure may not include silicone-based or PEG-based emulsifiers and preferably may not include synthetic emulsifiers.

The term "not include" means "substantially not include," and the "substantially" in this context means a content of up to 3% by weight based on the total weight of the composition. For example, the content accounting for "not include" may be 3% by weight or less, 2.5% by weight or less, 2% by weight or less, 1.5% by weight or less, 1% by weight or less, 0.7% by weight or less, 0.5% by weight or less, 0.3% by weight or less, 0.1% by weight or less, 0.06% by weight or less, 0.03% by weight or less, or 0.01% by weight or less.

### A. Polyglyceryl-based Emulsifier

The composition of the present disclosure includes a polyglyceryl-based emulsifier. In the present disclosure, the polyglyceryl-based emulsifier affects the pigment dispersibility and emulsion stability of the cosmetic composition.

The polyglyceryl-based emulsifier may include one or more selected from the group consisting of polyglyceryl penta-isostearate, polyglyceryl polyricinoleate, polyglyceryl-5 laurate, polyglyceryl-2 caprylate, and polyglyceryl-6 pentaoleate, with most preference for polyglyceryl penta-isostearate and polyglyceryl polyricinoleate.

The polyglyceryl-based emulsifier may be included in an amount of 0.1 to 20% by weight, based on the total weight of the composition. In the present disclosure, the polyglyceryl-based emulsifier may be included in an amount of 0.1% by weight or more, 1% by weight or more, 2% by weight or more, 5% by weight or more, or 10% by weight or more, and 20% by weight or less, 17% by weight or less, 15% by weight or less, 12% by weight or less, or 10% by weight or less. Given less than 0.1% by weight of the polyglyceryl-based emulsifier, the composition suffers from the disadvantage of poor pigment dispersibility and emulsion stability. More than 20% by weight of the polyglyceryl-based emulsifier makes the composition poor in spreadability and sensation of use.

### B. Oil-phase Thickener

The composition of the present disclosure includes an oil-phase thickener. So long as it is dispersed in the oil phase, any natural thickener known in the art may be employed. For example, at least one selected from the group consisting of hectorite and bentonite may be used.

The oil-phase thickener may be included in an amount of 0.1 to 5% by weight, based on the total weight of the composition. In the present disclosure, the oil-phase thickener may be included in an amount of 0.1% by weight or more, 1% by weight or more, 1.5% by weight or more, 2% by weight or more, or 2.5% by weight or more, and 5% by weight or less, 4.5% by weight or less, 4% by weight or less, 3.5% by weight or less, or 3% by weight or less. If the oil-phase thickener is included in an amount less than 0.1% by weight, the composition is too low in viscosity and suffers from poor pigment dispersibility and emulsion stability. If the amount exceeds 5% by weight, there may be problems with the viscosity of the composition being too high.

### C. Water-phase Thickener

The composition of the present disclosure includes a water-phase thickener. Any natural water-phase thickener known in the art may be employed as long as it is dispersed in the water phase. For instance, one or more selected from the group consisting of hectorite, bentonite, xanthan gum, magnesium silicate, magnesium aluminum silicate, lithium magnesium silicate, and magnesium sodium silicate may be used.

The water-phase thickener may be included in an amount of 0.1 to 5% by weight, based on the total weight of the composition. In the present disclosure, the water-phase thickener may be included in an amount of 0.1% by weight or more, 1% by weight or more, 1.5% by weight or more, 2% by weight or more, or 2.5% by weight or more, and 5% by weight or less, 4.5% by weight or less, 4% by weight or less, 3.5% by weight or less, or 3% by weight or less. Given less than 0.1% by weight of the water-phase thickener, the composition suffers from problems with low viscosity, pigment dispersibility, and emulsion stability. More than 5% by weight of the water-phase thickener may impart too high viscosity to the composition.

### D. Pigment

The composition of the present disclosure includes a pigment. Any pigment known in the field can be used. For instance, one or more selected from the group consisting of titanium dioxide, zinc oxide, yellow iron oxide, red iron oxide, and black iron oxide may be used.

In the present disclosure, the pigment may be coated. The coating material used for coating the pigment may be one or more selected from the group consisting of lauroyl lysine, lysine, sodium lauroyl glutamate, glutamate, and disodium stearoyl glutamate, with most preference for lauroyl lysine.

The pigment may be included in an amount of 0.1 to 50% by weight, based on the total weight of the composition. In the present disclosure, the pigment may be included in an amount of 0.1% by weight or more, 5% by weight or more, 100 by weight or more, 15% by weight or more, or 20% by weight or more, and 50% by weight or less, 45% by weight or less, 40% by weight or less, 35% by weight or less, or 30% by weight or less. If the pigment is included in an amount less than 0.1% by weight, the makeup effect may be poor. If the amount exceeds 50% by weight, the composition exhibits poor dispersibility and spreadability.

### E. Oil

The composition of the present disclosure may include oil. Any oil known in the field may be used. Preferably useful is at least one selected from the group consisting of hydrocarbon oil, ester oil, silicone oil, polyester, fatty acid and/or fatty alcohol, animal oil, vegetable oil, mineral oil, and mixtures thereof.

In the present disclosure, the oil may be included in an amount of 0.1 to 50% by weight, based on the total weigh of the composition, and preferably, 0.1% by weight or more, 1% by weight or more, 5% by weight or more, or 10% by weight or more, and 50% by weight or less, 40% by weight or less, or 30% by weight or less.

### F. Purified Water

The composition of the present disclosure may include purified water. In the present disclosure, the amount of purified water may be the balance that satisfies 100% of the composition. For example, purified water may be included in an amount of 0.1 to 50% by weight, or in another example, 1 to 45% by weight, 10 to 40% by weight, or 20 to 35% by weight, based on the total weight of the composition.

### G. Other Additives

The composition of the present disclosure may further include additives conventionally used according to use in the technical field to which the present disclosure belongs, within a range that does not impair the purpose and effect of the present disclosure. Preferably, the composition may include at least one selected from the group consisting of dispersants, polyols, preservatives, stabilizers, moisturizer, antioxidants, and fillers, but is not limited thereto.

In the present disclosure, the aforementioned other additives may be included in an amount of 0.1 to 20% by weight.

A better understanding of the present disclosure may be obtained through the following examples which are set forth to illustrate, but are not to construed to limit, the present disclosure.

### PREPARATION EXAMPLE 1: Preparation of Cosmetic Composition

Cosmetic compositions were prepared according to the configurations (weight%) shown in Table 1 below.

**TABLE 1**

| Component (wt%) | Ex. 1 | C. Ex. 1 | C. Ex. 2 | C. Ex. 3 | C. Ex. 4 | C. Ex. 5 | C. Ex. 6 | C. Ex. 7 |
|---|---|---|---|---|---|---|---|---|
| emulsifier - Polyglyceryl pentaisostearate | 5 | 5 | 5 | 5 | 5 | 5 | 5 | 5 |
| emulsifier - Polyglycerol polyricinoleate | 5 | 5 | 5 | 5 | 5 | 5 | 5 | 5 |
| Hectorite (water phase thickener) | 1 | - | 1 | 1 | 1 | - | 0.05 | 5 |
| Hectorite (oil phase thickener) | 1 | - | - | 0.05 | 5 | 1 | 1 | 1 |
| pigment - Iron oxide | 15 | 15 | 15 | 15 | 15 | 15 | 15 | 15 |
| polyol - Hexandiol | 1 | 1 | 1 | 1 | 1 | 1 | 1 | 1 |
| polyol - Glycerin | 5 | 5 | 5 | 5 | 5 | 5 | 5 | 5 |
| Polyhydroxystearic acid | 1 | 1 | 1 | 1 | 1 | 1 | 1 | 1 |
| Other additives | 1 | 1 | 1 | 1 | 1 | 1 | 1 | 1 |
| Purified water | To 100 | To 100 | To 100 | To 100 | To 100 | To 100 | To 100 | To 100 |
| Total | 100 | 100 | 100 | 100 | 100 | 100 | 100 | 100 |

Specifically, the components listed in Table 1 were weighed, and the emulsifier, oil-phase thickener, and polyhydroxystearic acid were heated and dissolved at 70°C and then added with the pigment. The mixture was stirred at 3500 rpm for 10 minutes using a homogenizer to prepare the oil phase. Purified water, polyol, water-phase thickener, and other additives were stirred together at 3500 rpm for 10 minutes using a homogenizer to prepare the water phase. The oil phase and water phase were mixed, and the mixture was stirred at 6000 rpm for 10 minutes using a homogenizer to prepare the cosmetic composition.

### Viscosity Measurement

The compositions in the Examples and Comparative Examples were measured for viscosity.

The viscosity of each composition prepared in Example 1 and Comparative Examples 1 to 7 was measured using a Brookfield viscometer under conditions of 64 spindle, 12 rpm, 1 minute, and 25°C. The results are given in Table 2 below.

**TABLE 2**

| | Ex. 1 | C. Ex. 1 | C. Ex. 2 | C. Ex. 3 | C. Ex. 4 | C. Ex. 5 | C. Ex. 6 | C. Ex. 7 |
|---|---|---|---|---|---|---|---|---|
| Viscosity (cps) | 10,000 | - | 3,000 | 3,000 | 35,000 | 2,000 | 2,000 | 40,000 |

As shown in Table 2, the composition of Example 1 had a viscosity of 10,000 cps, which is desirable for a foundation cosmetic composition. In contrast, the composition of Comparative Example 1, which did not include any water-phase or oil-phase thickener, had an unmeasurable viscosity. Low viscosities were detected in Comparative Example 2, which included only a water-phase thickener, Comparative Example 3, with a low content of oil-phase thickener, Comparative Example 5, which included only an oil-phase thickener, and Comparative Example 6, with a low content of water-phase thickener. Meanwhile, Comparative Example 4 with a high content of oil-phase thickener and Comparative Example 7 with a high content of water-phase thickener had viscosities that were too high, making the compositions undesirable.

The viscosity measurement results indicate that the cosmetic composition of the present disclosure provides a foundation cosmetic composition with a desirable viscosity.

### Formulation Stability Test

A formulation stability test was conducted on the compositions of the Examples and Comparative Examples.

The test involved storing each composition prepared in Example 1 and Comparative Examples 1 to 7 in a temperature- and humidity-controlled chamber at 25°C for 12 weeks, and observing formulation changes visually. The results after 12 weeks are shown in Table 3 below.

**TABLE 3**

| | Ex. 1 | C. Ex. 1 | C. Ex. 2 | C. Ex. 3 | C. Ex. 4 | C. Ex. 5 | C. Ex. 6 | C. Ex. 7 |
|---|---|---|---|---|---|---|---|---|
| Formulat ion state | Stab le | Phase separat ion | Phase separat ion | Phase separat ion | Stab le | Phase separat ion | Phase separat ion | Stab le |

As shown in Table 3, Example 1 remained stable over the 12-week period. In contrast, Comparative Example 1, which did not contain any water-phase or oil-phase thickener, were observed to undergo phase separation. In addition, phase separation was also observed in Comparative Examples 2 and 5, which included only a water-phase or oil-phase thickener, and in Comparative Examples 3 and 6, which had low contents of either thickener.

From the formulation stability test, it was confirmed that the cosmetic composition of the present disclosure maintained its stability without phase separation for 12 weeks.

### Test for Sensation of Use

A test for sensation of use was conducted on the compositions of the Examples and Comparative Examples.

In the test, 20 women aged 20 to 40 used each composition prepared in Example 1 and Comparative Examples 4 and 7 and then evaluated the sensation of use based on four criteria: stiffness during application, greasiness, darkening, and color durability. Each criterion was scored from 1 to 10, and the average score was calculated from the total scores of the 20 participants. The results are summarized in Table 4 below. Higher scores accounted for less appearance of symptoms in stiffness, greasiness, and darkening, and color durability was given a high score as the duration lasted long.

### - Evaluation Criteria

8 points and above: Very good (⊚), 6 to 8 points: Good (o), 3 to 6 points: Fair (△), Below 3 points: Poor (×)

**TABLE 4**

| Test Criteria | Ex. 1 | C. Ex. 4 | C. Ex. 7 |
|---|---|---|---|
| Stiffness | ⊚ | × | × |
| Greasiness | ⊚ | △ | △ |
| Darkening | ⊚ | × | × |
| Color durability | ⊚ | × | × |

As shown in Table 4, Example 1 received excellent scores for sensation of use in all criteria. In contrast, Comparative Examples 4 and 7 received low scores in all criteria.

From the test for sensation of use, it was confirmed that the cosmetic composition of the present disclosure provided excellent sensation of use, with no stiffness or greasiness during application, no darkening, and excellent color durability.

### PREPARATION EXAMPLE 2: Preparation of Cosmetic Composition

Cosmetic compositions were prepared according to the configurations (weight%) shown in Table 5 below. The specific preparation method is the same as in Example 1.

**TABLE 5**

| Component (wt%) | Ex. 2 | Ex. 3 | C. Ex. 8 | C. Ex. 9 | C. Ex. 10 |
|---|---|---|---|---|---|
| emulsifier - Polyglyceryl pentaisostearate | 5 | 3 | 25 | - | - |
| emulsifier - Polyglycerol polyricinoleate | 3 | 5 | - | 25 | - |
| Sorbitan isostearate | - | - | - | - | 25 |
| Hectorite (oil phase thickener) | 1 | 1 | 1 | 1 | 1 |
| Hectorite (water phase thickener) | 1 | 1 | 1 | 1 | 1 |
| Pigment - Iron oxide | 15 | 15 | 15 | 15 | 15 |
| Polyol - Hexandiol | 1 | 1 | 1 | 1 | 1 |
| Polyol - Glycerin | 5 | 5 | 5 | 5 | 5 |
| Polyhydroxystearic acid | 1 | 1 | 1 | 1 | 1 |
| Other additives | 1 | 1 | 1 | 1 | 1 |
| Purified water | To 100 | To 100 | To 100 | To 100 | To 100 |
| Total | 100 | 100 | 100 | 100 | 100 |

### Viscosity Measurement

The compositions in the Examples and Comparative Examples were measured for viscosity.

The viscosity of each composition prepared in Examples 2 and 3 and Comparative Examples 8 to 10 was measured using a Brookfield viscometer under conditions of 64 spindle, 12 rpm, 1 minute, and 25°C. The results are given in Table 6, below.

**TABLE 6**

| | Ex. 2 | Ex. 3 | C. Ex. 8 | C. Ex. 9 | C. Ex. 10 |
|---|---|---|---|---|---|
| Viscosity (cps) | 10,000 | 10,000 | 45,000 | 45,000 | - |

As shown in Table 6, Examples 2 and 3 had a viscosity of 10,000 cps, which is desirable for a foundation cosmetic composition. In contrast, Comparative Examples 8 and 9, with high contents of polyglyceryl-based emulsifier, were measured to have high viscosities. Comparative Example 10, which included sorbitan isostearate, had an unmeasurable viscosity.

The viscosity measurement results indicate that the cosmetic composition of the present disclosure provides a foundation cosmetic composition with a desirable viscosity.

### Formulation Stability Test

A formulation stability test was conducted on the compositions of the Examples and Comparative Examples.

In this test, each composition prepared in Examples 2 and 3 and Comparative Examples 8 to 10 was stored in a temperature- and humidity-controlled chamber at 25°C for 12 weeks, and observed for formulation changes visually. The results after 12 weeks are shown in Table 7 below.

**TABLE 7**

| | Ex. 2 | Ex. 3 | C. Ex. 8 | C. Ex. 9 | C. Ex. 10 |
|---|---|---|---|---|---|
| Formulation state | Stable | Stable | Stable | Stable | Phase separation |

As shown in Table 7, Examples 2 and 3 and Comparative Examples 8 and 9, which all included polyglyceryl-based emulsifiers, remained stable over the 12-week period. In contrast, Comparative Example 10, which included sorbitan isostearate, showed phase separation.

From the formulation stability test, it was confirmed that the cosmetic composition of the present disclosure maintained its stability without phase separation for 12 weeks.

### Test for Sensation of Use

A test for sensation of use was conducted on the compositions of the Examples and Comparative Examples.

In the test, 20 women aged 20 to 40 used each composition prepared in Examples 2 and 3 and Comparative Examples 8 and 9 and then evaluated the sensation of use based on four criteria: stiffness during application, greasiness, darkening, and color durability. Each criterion was scored from 1 to 10, and the average score was calculated from the total scores of the 20 participants. The results are summarized in Table 8 below. Higher scores accounted for less appearance of symptoms in stiffness, greasiness, and darkening, and color durability was given a high score as the duration lasted long.

### - Evaluation Criteria

8 points and above: Very good (⊚), 6 to 8 points: Good (o), 3 to 6 points: Fair (Δ), Below 3 points: Poor (x)

**TABLE 8**

| Test Criteria | Ex. 2 | Ex. 3 | C. Ex. 8 | C. Ex. 9 |
|---|---|---|---|---|
| Stiffness | ⊚ | ⊚ | x | x |
| Greasiness | ⊚ | ⊚ | △ | △ |
| Darkening | ⊚ | ⊚ | x | x |
| Color durability | ⊚ | ⊚ | x | x |

As shown in Table 8, Examples 2 and 3 received excellent scores for sensation of use in all criteria. In contrast, Comparative Examples 8 and 9 received low scores in all criteria.

From the test for sensation of use, it was confirmed that the cosmetic composition of the present disclosure provided excellent sensation of use, with no stiffness or greasiness during application, no darkening, and excellent color durability.

## Claims

1. A cosmetic composition, comprising a polyglyceryl-based emulsifier, an oil-phase thickener, a water-phase thickener, and a pigment.

2. The cosmetic composition of claim 1, wherein the polyglyceryl-based emulsifier comprises at least one selected from the group consisting of polyglyceryl pentaisostearate, polyglyceryl polyricinoleate, polyglyceryl-5 laurate, polyglyceryl-2 caprylate, and polyglyceryl-6 pentaoleate.

3. The cosmetic composition of claim 1, wherein the polyglyceryl-based emulsifier is included in an amount of 0.1 to 20% by weight, based on the total weight of the total composition.

4. The cosmetic composition of claim 1, wherein the oil-phase thickener comprises at least one selected from the group consisting of hectorite and bentonite.

5. The cosmetic composition of claim 1, wherein the oil-phase thickener is included in an amount of 0.1 to 5% by weight, based on the total weight of the composition.

6. The cosmetic composition of claim 1, wherein the water-phase thickener comprises at least one selected from the group consisting of hectorite, bentonite, xanthan gum, magnesium silicate, magnesium aluminum silicate, lithium magnesium silicate, and magnesium sodium silicate.

7. The cosmetic composition of claim 1, wherein the water-phase thickener is included in an amount of 0.1 to 5% by weight, based on the total weight of the composition.

8. The cosmetic composition of claim 1, wherein the pigment comprises at least one selected from the group consisting of titanium dioxide, zinc oxide, yellow iron oxide, red iron oxide, and black iron oxide.

9. The cosmetic composition of claim 1, wherein the pigment is included in an amount of 0.1 to 50% by weight, based on the total weight of the composition.

10. The cosmetic composition of claim 1, wherein the pigment is coated with at least one selected from the group consisting of lauroyl lysine, lysine, sodium lauroyl glutamate, glutamate, and disodium stearoyl glutamate.

11. The cosmetic composition of claim 1, wherein the composition has a viscosity of 3,000 to 30,000 cps.

12. The cosmetic composition of claim 1, wherein the composition has a hardness of 10 to 30 dyne/cm².
